# EUROPÄISCHE PATENTSCHRIFT

(11) **EP 2 255 838 B1**
(45) Veröffentlichungstag und Bekanntmachung des Hinweises auf die Patenterteilung: **05.07.2017**
(21) Anmeldenummer: 10163769.2
(22) Anmeldetag: 25.05.2010
(51) Int. Cl.: A61M 1/02, A61M 1/34, A61M 1/36

(54) **VERFAHREN UND VORRICHTUNG ZUM AUFTRENNEN VON VOLLBLUT**
PROCESS AND DEVICE FOR SEPARATING WHOLE BLOOD
PROCÉDÉ ET DISPOSITIF POUR SÉPARER DU SANG ENTIER

(30) Priorität: 26.05.2009 DE 102009022605
(43) Veröffentlichungstag der Anmeldung: 01.12.2010
(73) Patentinhaber: Lmb Lab med Blutbank Technologie GmbH, 85445 Schwaig (DE)
(72) Erfinder: Jentsch, Klaus, 85445, Schwaig (DE)
(74) Vertreter: Winter, Brandl, Fürniss, Hübner, Röss, Kaiser, Polte - Partnerschaft mbB

(56) Entgegenhaltungen:
- WO-A1-00/23140
- WO-A1-00/62840
- WO-A2-2004/050145

## Beschreibung

Die vorliegende Erfindung betrifft ein Verfahren zum Auftrennen von Vollblut in eine im Wesentlichen zellfreie Plasmafraktion und eine Erythrozyten/Plasma-Suspension, sowie eine Vorrichtung zur Durchführung dieses Verfahrens.

Die vorliegende Erfindung wird durch den Gegenstand der unabhängigen Ansprüche definiert. Die Beschreibung dient der Veranschaulichung des Erfindungsgegenstands und zeigt mögliche Anwendungsgebiete auf.

Verfahren und Vorrichtungen zum Separieren oder Auftrennen von Vollblut in bestimmte Fraktionen sind aus dem Stand der Technik in unterschiedlichen Ausprägungen bekannt. Verwiesen werden darf beispielsweise auf die DE 102 39 658 A1, die EP 1 089 778 B1 oder das DE 295 16 471 U1.

Filtervorrichtungen, mit welchen Vollblut in verschiedene Fraktionen aufteilbar ist, sind unter anderem aus der EP 0 893 130 B1, der EP 0 868 208 B1 oder dem DE 200 14 311 U1 bekannt.

Auch die Druckschrift WO 2004/050145 A2 offenbart eine Vorrichtung zum Auftrennen von Vollblut in mehrere Fraktionen, mit einem Aufnahmebeutel für Vollblut, einer Filtereinheit, einem Auffangbeutel für Plasma und einem Auffangbeutel für Erythrozytenkonzentrat. Diese Komponenten sind miteinander über Leitungen verbunden. Zum Fraktionieren von Blut bewegt ein Benutzer den Aufnahmebeutel mit dem Vollblut derart, beispielsweise nach oben, dass durch die Schwerkraft in dem Aufnahmebeutel enthaltenes Blut durch die Filtereinheit und nach Fraktionen separiert in die jeweiligen Auffangbeutel fließt. Danach bewegt der Benutzer beispielsweise den Auffangbeutel mit dem Erythrozytenkonzentrat derart, beispielsweise nach oben, dass das in dem Auffangbeutel enthaltene Erythrozytenkonzentrat durch dieselbe Leitung wieder durch die Filtereinheit - diesmal in entgegengesetzter Richtung- in den Aufnahmebeutel zurück

fließt. Auf diese Weise kann das Blut durch die Filtereinheit wiederholt hin-undher-bewegt und somit in Fraktionen separiert werden.

Auch die Druckschrift WO 00/62840 offenbart eine Vorrichtung zum Separieren von Blut in verschieden Fraktionen mit einem Aufnahmebeutel, einer Filtereinheit und
jeweils einem Auffangbeutel für Plasma und Erythrozytenkonzentrat. Um den Druck in der Filtereinheit einstellen zu können, weist diese Vorrichtung stromabwärts der Filtereinheit eine Klemme auf, mit der ein definierter Rückstau in der Filtereinheit erzeugt werden kann.

Die EP 1 567 210 B1 beschreibt ein Verfahren und eine Vorrichtung zum Separieren von Vollblut unter Schwerkraft in ein Erythrozytenkonzentrat und ein zellfreies oder thrombozytenhaltiges Plasma. Hierbei wird das Vollblut unter Schwerkraft durch Filtration in ein erstes Erythrozyten-Konzentrat und in eine erste, thrombozytenhaltige Plasmafraktion aufgetrennt. Nach diesem ersten Trennvorgang wird das Erythrozyten-Konzentrat erneut einer Plasmafiltration unter Erhöhung des Hämatokrits unterworfen, wobei diese erneute Plasmafiltration gegebenenfalls ein drittes Mal durchgeführt werden kann. Das letztendlich nach den Filtrationsschritten erhaltene Erythrozyten-Konzentrat wird mit einer Additivlösung versehen und durch einen letzten Filtrationsschritt von Mikroaggregaten und Leukozyten befreit.

Weiterhin offenbart die Druckschrift WO 00/23140 eine technisch recht komplexe Vorrichtung zur Blutverarbeitung mit mehreren peristaltischen Pumpen, Drucksensoren, einer Vielzahl von Ventilen und einer Steuerungseinrichtung.

Der leitungstechnische und damit steuerungs- und überwachungstechnische Aufwand bei der EP 1 567 210 B1 und der WO 00/23140 ist erheblich; die vorliegende Erfindung hat es sich zur Aufgabe gemacht, diesen Aufwand zu verringern, sodass beispielsweise die Anzahl oder Menge von Leitungen, die nach der erfolgten Auftrennung einer Charge zu entsorgen sind, weil sie kontaminiert sind, ebenfalls entsprechend verringert werden kann.

Der Gegenstand der vorliegenden Erfindung zeichnet im Wesentlichen dadurch aus, dass bei einem Verfahren zum Auftrennen von Vollblut in Fraktionen, bei dem Vollblut unter Schwerkrafteinfluss durch eine Hohlfasermembran-Filtereinheit geleitet wird, um wenigstens zwei Fraktionen zu erhalten, dann eine der Fraktionen rezirkulierend an den

Einlass der Hohlfasermembran-Filtereinheit zurückgeführt wird, um diese noch wenigstens einmal erneut zu durchlaufen.

Eine besonders bevorzugte Ausgestaltung der Erfindung befasst sich mit einem Verfahren, bei dem das Vollblut in eine im Wesentlichen zellfreie Plasmafraktion und eine Erythrozyten/Plasma-Suspension aufgetrennt wird, in dem aus dem Vollblut zunächst Thrombozyten und Leukozyten unter Verbleib von Erythrozyten und Plasma in Suspension abgetrennt werden. Nach Erhalt der Plasmafraktion und der Erythrozyten/Plasma-Suspension mittels der Hohlfasermembran-Filtereinheit wird die Erythrozyten/Plasma-Suspension rezirkulierend an den Einlass der Hohlfasermembran-Filtereinheit zurückgeführt, um diese noch wenigstens einmal erneut zu durchlaufen.

Die wiederholte rezirkulierende Rückführung der Erythrozyten/Plasma-Suspension an den Einlass der Hohlfasermembran-Filtereinheit kann hierbei bevorzugt so oft erfolgen, bis eine Fließfähigkeitsgrenze der eingedickten Erythrozyten im Restplasma erreicht ist.

Besagte Fließfähigkeitsgrenze entspricht hierbei im Wesentlichen einem Hämatokrit von etwa 90%.

Die rezirkulierend rückgeführte Erythrozyten/Plasma-Suspension kann vor dem erneuten Durchlaufen der Filtereinheit mit einer Nähr- und/oder Spüllösung unter sterilen Bedingungen aufgeschwemmt werden. Hiermit kann ein weiterer Anteil an störenden Komponenten entfernt werden, da besagte störende Komponenten zusammen mit der Spüllösung in der Filtereinheit entfernt werden, also aus der Erythrozyten/Plasma-Suspension abgezogen werden.

Die Aufschwemmung mit der Nähr- und/oder Spüllösung kann hierbei bevorzugt vor jedem erneuten Durchlaufen der Filtereinheit erfolgen, sodass im Zuge einer jeden Rezirkulation der Erythrozyten/Plasma-Suspension diese sukzessive von verbleibenden Plasma- und Metabolitenanteilen befreit wird.

Es kann bevorzugt oder falls die besonderen Auftrennvorgaben es nötig machen, der Erythrozyten/Plasma-Suspension zumindest vor einem Durchlaufen der Filtereinheit wenigstens eine, die zellulären Eigenschaften der Suspension in gewünschter Weise verändernde Substanz zugefügt werden, welche dann in der Filtereinheit nachfolgend wieder entfernt werden kann.

Als ein zentrales Sammelorgan für beispielsweise additive Lösungen, aber auch das Vollblut, welches gegebenenfalls bereits leukozytenbefreit sein kann und die Erythrozyten/Plasma-Suspension welche nach Durchlauf der Filtereinheit gewonnen und an besagte Filtereinheit zurückgeführt werden soll, ist bevorzugt ein zentraler Behandlungsbeutel vorgesehen, welcher als eine Art Vorlegebehälter der Filtereinheit vorgeschaltet ist.

Eine Vorrichtung zur Durchführung des erfindungsgemäßen Verfahrens; bei dem das Vollblut in die im Wesentlichen zellfreie Plasmafraktion und die Erythrozyten/Plasma-Suspension aufgetrennt wird, umfasst im Wesentlichen einen Entnahmebeutel zur Aufnahme einer Vollblutkonserve; einen dem Entnahmebeutel stromabwärts nachgeschalteten Leukozytenfilter; einen dem Leukozytenfilter stromabwärts nachgeschalteten Verarbeitungsbeutel; eine dem Verarbeitungsbeutel stromabwärts nachgeschaltete Hohlfasermembran-Filtereinheit; und der Hohlfasermembran-Filtereinheit stromabwärts nachgeschaltete Sammelbeutel zumindest für eine Erythrozyten/Plasma-Suspension und eine von der Filtereinheit abgetrennte Plasmafraktion, wobei der Sammelbeutel für die Erythrozyten/Plasma-Suspension eine Fluidverbindung aufweist, welche die Erythrozyten/Plasma-Suspension aus ihrem Sammelbeutel stromaufwärts zu den Verarbeitungsbeutel zurückführt und damit rezirkuliert.

Weitere Einzelheiten, Aspekte und Vorteile der vorliegenden Erfindung ergeben sich besser aus der Beschreibung einer Ausführungsform an Hand der Zeichnung.

Es zeigt:
Fig. 1 bis Fig. 5 jeweils den Aufbau einer erfindungsgemäßen Vorrichtung zum Auftrennen von Vollblut in schematischer Darstellung und in unterschiedlichen Ablaufschritten des erfindungsgemäßen Verfahrens;
Fig. 6 eine Modifikation der Vorrichtung gemäß der Fig. 1 bis 5; und
Fig. 7 eine Fig. 6 entsprechende Darstellung in einem anderen Ablaufschritt.

Die nachfolgende Beschreibung der vorliegenden Erfindung erfolgt anhand des praxisorientierten Ausführungsbeispiels einer Vorrichtung, bei der Vollblut in eine im Wesentlichen zellfreie Plasmafraktion und eine Erythrozyten/Plas-ma-Suspension aufgetrennt wird.

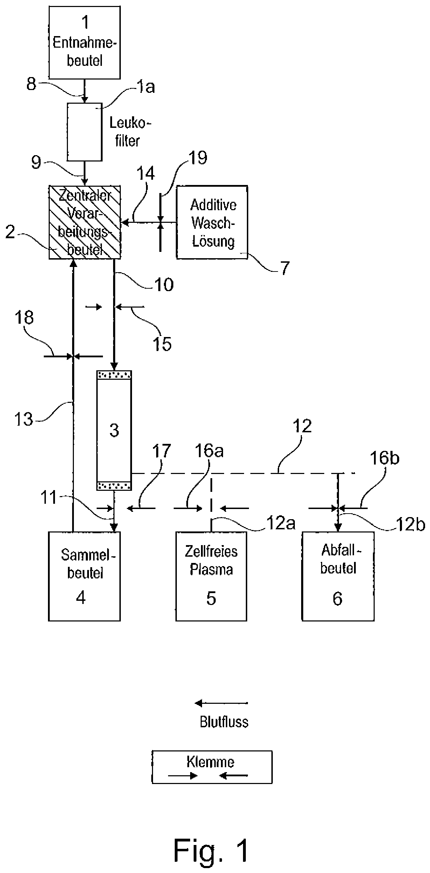

Eine erfindungsgemäße Vorrichtung umfasst hierzu gemäß der schematischen Darstellung in der Zeichnung und hier insbesondere gemäß Fig. 1 im Wesentlichen einen Entnahmebeutel 1 für Vollblut, einen dem Entnahmebeutel 1 stromabwärts nachgeschalteten Leukozytenfilter 1 a, sowie einen dem Leukozytenfilter wiederum stromabwärts nachgeschalteten zentralen Verarbeitungsbeutel 2.

Dem Verarbeitungsbeutel 2 nachgeschaltet ist eine Filtereinheit 3, insbesondere eine Hohlfasermembran-Filtereinheit, welche zum Auftrennen oder Separieren des durch den Leukozytenfilter 1 a von Leukozyten befreiten Bluts aus dem Entnahmebeutel 1 beziehungsweise dem zentralen Verarbeitungsbeutel 2 dient. Der Filtereinheit 3 ist stromabwärts eine Mehrzahl von Aufnahme- oder Sammelbeuteln 4 bis 6 nachgeschaltet, wobei der Sammelbeutel 4 zur Aufnahme einer Erythrozyten/Plasma-Suspension aus der Filtereinheit 3 dient, der Sammelbeutel 5 zur Aufnahme von im Wesentlichen zellfreiem Plasma aus der Filtereinheit 3 dient und der Sammelbeutel 6 als Abfallbeutel für sonstige Bestandteile dient, die von der Filtereinheit 3 abgetrennt werden.

Zwischen den jeweiligen Komponenten 1 bis 7 befinden sich Schlauchleitungen 8 bis 14, wobei sich in den Schlauchleitungen 10, 11, 12a, 12b, 13 und 14 Schlauchklemmen 15 bis 19 befinden, mit denen diese Leitungen unabhängig von einander in einen offenen oder versperrten Zustand steuerbar sind.

Weiterhin unter Bezugnahme auf die in Zeichnung sei nachfolgend der Ablauf eines erfindungsgemäßen Separations- oder Auftrennverfahrens mittels der erfindungsgemäßen Vorrichtung näher erläutert.

Zur Herstellung von humanem Erythrozytenkonzentrat und Plasma wird von einem geeigneten Spender eine Menge von 450 bis 500ml Vollblut entnommen, welches in dem Blutbeutel 1 zusammen mit einer gerinnungshemmenden Nährlösung versetzt aufbewahrt wird. Der so erhaltene Vollblutbeutel 1 wird in die erfindungsgemäße Auftrennvorrichtung eingebracht, das heißt der Entnahmebeutel 1 wird mittels der Leitung 8 mit dem Eingang des Leukozytenfilters 1a verbunden, sodass das Vollblut aus dem Beutel 1 unter Schwerkrafteinfluss den Leukozytenfilter 1a durchströmt und hier von leukozytären und thrombozytären Bestandteilen gereinigt wird. Es passiert somit eine Suspension aus Erythrozyten, Plasma und Stabilisatoren den Filter 1 a und gelangt in den zentralen Verarbeitungsbeutel 2.

In diesem zentralen Verarbeitungsbeutel 2 kann sich die aus dem Leukozytenfilter 1 a gewonnene Suspension von den applizierten Verformungskräften erholen, sodass sich wieder ein Gleichgewicht zwischen intra- und extrazellulären Bestandteilen einstellen kann. Nach einer gründlichen Durchmischung wird dann die Suspension über die Leitung 10 und unter Schwerkrafteinfluss der Filtereinheit 3 zugeführt, welche insbesondere eine Hohlfasermembran-Filtereinheit ist. Der Druck auf die Hohlfasermembranen wird durch einen entsprechend geringen Höhenunterschied zwischen der Aufhängungshöhe des zentralen Verarbeitungsbeutels 2 und dem Einlauf des Filters 3 niedrig gehalten. Die daraus resultierende geringe Fließgeschwindigkeit schont die durchfließenden Erythrozyten, sodass diese nicht an den Membranen der Filtereinheit 3 verletzt werden.

Weiterhin wird der Höhenunterschied zwischen dem Ausgang der Filtereinheit 3 und dem sich stromabwärts befindlichen Sammelbeutel 4 niedrig gehalten, damit der entsprechend niedrig eingestellte Fließunterdruck ebenfalls keine Schäden an dem Bestandteilen anrichten kann.

Nach dem Durchgang durch die Filtereinheit 3 sammelt sich in dem Sammelbeutel 4 eine Erythrozyten/Plasma-Suspension mit einem verminderten Anteil an Plasma und gerinnungshemmender Nährlösung und im Sammelbeutel 5 sammelt sich im Wesentlichen zellfreies Plasma.

Bei diesem ersten Separationsschritt sind die Schlauchklemmen 15, 16a und 17 in den Leitungen 10, 11, und 12a geöffnet.

Nach diesem ersten Separationsschritt werden die soeben genannten Schlauchklemmen geschlossen und die Klemme 18 in der Leitung 13 zwischen dem Sammelbeutel 4 und dem zentralen Verarbeitungsbeutel 2 wird geöffnet. Somit wird der Inhalt des Sammelbeutels 4 wieder in den Verarbeitungsbeutel 2 zurückgeführt und durchfließt dann erneut in der gleichen Richtung wie beim vorherigen Separationsschritt die Hohlfasermembran-Filtereinheit 3. Hierdurch wird erneut Plasma in dem Sammelbeutel 5 abgeschieden und die weiter eingedickte Erythrozyten/Plasma-Suspension in den Sammelbeutel 4 überführt.

Die aus der Filtereinheit 3 gewonnene Erythrozyten/Plasma-Suspension wird somit rezirkulierend oder im Kreislauf solange durch die Filtereinheit 3 geführt, wie die Erythrozyten in der so gewonnen Suspension fließfähig bleiben.

Da eine möglichst reine Präparation von Körperzellen angestrebt ist und die Erythrozyten nach der Entfernung des Plasmas in der gerinnungshemmenden Lösung mit Plasma und weiteren Metaboliten aufgeschwemmt sind, wird bevorzugt im gleichen geschlossenen System ein Spülvorgang angewendet. Die Erythrozyten werden mit einer geeigneten Spül- und/oder Nährlösung aufgeschwemmt und erneut durch die Filtereinheit geleitet. Die Spül- und/oder Nährlösung wird in der Filtereinheit auf die andere Filterseite geführt und danach in dem Sammelbeutel 6 aufgefangen. Die Erythrozyten sammeln sich erneut wieder unterhalb der Filtereinheit 3 im Sammelbeutel 4. Über die Leitung 13 können sie von dort aus erneut in den zentralen Verarbeitungsbeutel 2 überführt werden und erneut mit Spül- und/oder Nährlösungen oder dergleichen aus dem Behälter oder Beutel 7 aufgeschwemmt werden. Auch dieser Vorgang kann im Zuge des Rezirkulationsverfahrens theoretisch beliebig oft wiederholt werden.

Auf diese Weise werden letztendlich die Erythrozyten weitestgehend von Plasmaanteilen befreit und stehen als im Wesentlichen reines Arzneimittel zur Verfügung.

Es kann notwendig oder erwünscht sein, den zellulären Blutprodukten (Erythrozyten, Thrombozyten, Stammzellen, T-Lymphozyten, B-Lymphozyten, Granolozyten und Plasmazellen) wirksame Substanzen oder Verfahrensschritte zuzufügen, welche aber vor dem letztendlichen Gebrauch der gewonnenen Erythrozyten wieder entfernt werden müssen.

Es kann notwendig oder erwünscht sein, die Zellen Thrombozyten, Stammzellen, T-Lymphozyten, B-Lymphozyten, Granulozyten oder Plasmazellen nicht durch Filtration zu entfernen, sondern zunächst zusammen mit den Erythrozyten zu belassen, um wirksame Substanzen oder Verfahrungsschritte zuzufügen, welche nach dieser Behandlung gegebenenfalls wieder entfernt werden.

Es ist aber auch möglich, Präparationen bestimmter Zellen, wie Thrombozytenkonzentrate oder Thrombozytenhochkonzentrate, Nabelschnurblut, Stammzellpräparationen, Aufbereitungen von T-, B-Lymphozyten, Killerzellen, Makrophagen, Tumorzellen menschlichen oder tierischen Ursprungs an das Hohlfasersystem anzuschließen und gezielt chemische, physikalische, immunologische Kräfte einwirken zu lassen und gegebenenfalls die Substanzen durch Spül- und Verdünnungsvorgänge zu entfernen oder zu neutralisieren.

Auf Grund der Hohlfasermembran-Filtereinheit 3 ist es möglich, diese Substanzen erst gezielt zuzufügen, einwirken zu lassen (insbesondere im zentralen Verarbeitungsbeutel 2) und/oder zusätzliche chemische oder physikalische Kräfte zu applizieren (Mischen, Zentrifugieren oder dergleichen).

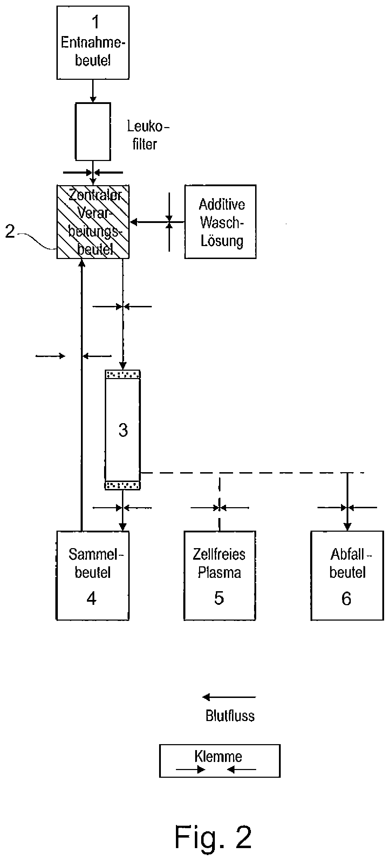

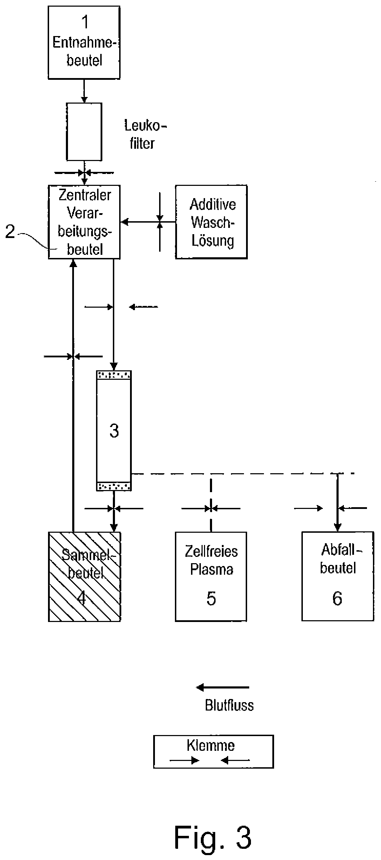

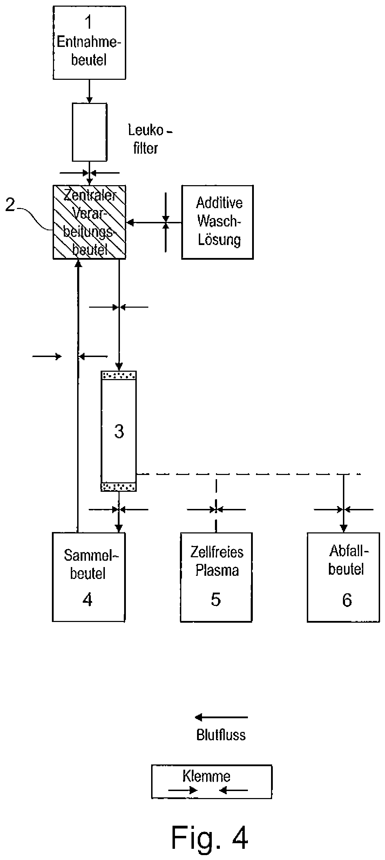

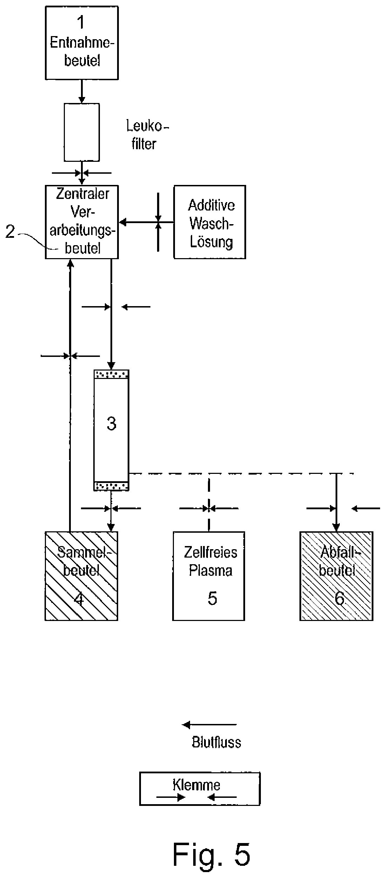

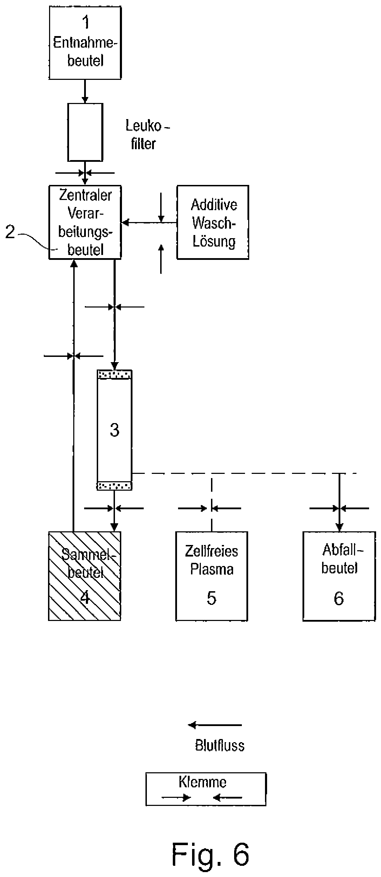

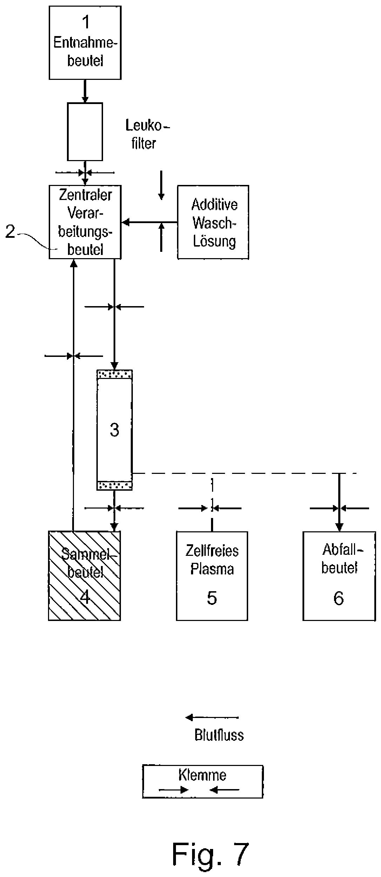

Die in den Fig. 1 bis 7 dargestellten wesentlichen Verfahrensschritte sind im Licht der obigen Erläuterungen, sowie aus den jeweiligen Offen- und Geschlossenstellungen der Schlauchklemmen in den jeweiligen Leitungen selbsterläuternd, sodass auf eine ausführliche Beschreibung dieser Verfahrensschritte hier verzichtet werden kann.

Es sei nochmals darauf hingewiesen, dass voran stehend ein Ausführungsbeispiel einer Vorrichtung beschrieben wurde, bei der Vollblut in eine im Wesentlichen zellfreie Plasmafraktion und eine Erythrozyten/Plasma-Suspension aufgetrennt wird. Dies stellt jedoch nur eine von vielen möglichen Ausgestaltungsformen der vorliegenden Erfindung dar.

## Patentansprüche

1. Verfahren zum Auftrennen von Vollblut in wenigstens zwei Fraktionen unter Schwerkrafteinfluss mit Hilfe einer Hohlfasermembran-Filtereinheit (3), mit den Schritten:
- Abtrennen von Thrombozyten und Leukozyten aus dem in einem Entnahmebeutel (1) enthaltenen Vollblut mittels eines Leukozytenfilters unter Verbleib von Erythrozyten und Plasma in Suspension,
- Leiten der Erythrozyten/Plasma-Suspension in einen Verarbeitungsbeutel (2),
- Abtrennen von zellfreiem Plasma von der Erythrozyten-/Plasma-Suspension in einem ersten Separationsschritt, indem diese von dem Verarbeitungsbeutel (2) durch die Hohlfasermembran-Filtereinheit (3) in Sammelbeutel (4,5,6) geleitet wird, Aufschwemmen der so gewonnenen Erythrozyten/Plasma-Suspension in dem Verarbeitungsbeutel (2) mit einer Nähr- und/oder Spüllösung unter sterilen Bedingungen (7, 14, 19);
- Abtrennen von zellfreiem Plasma von der aufgeschwemmten Erythrozyten/Plasma-Suspension in einem zweiten Separationsschritt, indem diese im Kreislauf von dem zumindest einen Sammelbeutel (4) für die Erythrozyten/Plasma-Suspension über eine Fluidverbindung (13) in den Verarbeitungsbeutel (2) und danach erneut durch die Hohlfasermembran-Filtereinheit (3) geleitet wird, wobei
- die Erythrozyten/Plasma-Suspension die Hohlfasermembran-Filtereinheit (3) in beiden Separationsschritten in der gleichen Richtung durchströmt.

2. Verfahren nach Anspruch 1, **dadurch gekennzeichnet, dass** die gegebenenfalls wiederholte rezirkulierende Rückführung (13, 18, 2) der Erythrozyten/Plasma-Suspension an den Einlass der Hohlfasermembran-Filtereinheit (3) so oft erfolgt, bis eine Fließfähigkeitsgrenze der eingedickten Erythrozyten im Restplasma erreicht ist.

3. Verfahren nach Anspruch 2, **dadurch gekennzeichnet, dass** die Fließfähigkeitsgrenze im Wesentlichen einem Hämatokrit von etwa 90% entspricht.

4. Verfahren nach Anspruch 1, **dadurch gekennzeichnet, dass** die Aufschwemmung mit der Nähr- und/oder Spüllösung vor jedem erneuten Durchlaufen der Hohlfasermembran-Filtereinheit (3) erfolgt.

5. Verfahren nach einem der Ansprüche 1 bis 4, **dadurch gekennzeichnet, dass** der Erythrozyten/Plasma-Suspension zumindest vor einem Durchlaufen der Hohlfasermembran-Filtereinheit (3) wenigstens eine, die zellulären Eigenschaften der Erythrozyten/Plasma-Suspension in gewünschter Weise verändernde Substanz zugefügt wird.

6. Verfahren nach Anspruch 5, **dadurch gekennzeichnet, dass** die wenigstens eine, die zellulären Eigenschaften der Erythrozyten/Plasma-Suspension verändernde Substanz in der Hohlfasermembran-Filtereinheit (3) wieder entfernt wird.

7. Verfahren nach einem der Ansprüche 1 bis 6, **dadurch gekennzeichnet, dass** die Zurückführung und gegebenenfalls Behandlung der im ersten Durchlauf gewonnenen Erythrozyten/Plasma-Suspension in einem zentralen Behandlungsbeutel (2) erfolgt, welcher der Hohlfasermembran-Filtereinheit (3) vorgeschaltet ist.

8. Zur Durchführung des Verfahrens nach einem oder mehreren der Ansprüche 1 bis 7 vorgesehene Vorrichtung, aufweisend:
einen Entnahmebeutel (1) zur Aufnahme einer Vollblutkonserve;
einen dem Entnahmebeutel (1) stromabwärts nachgeschalteten Leukozytenfilter (1a);
einen dem Leukozytenfilter (1a) stromabwärts nachgeschalteten Verarbeitungsbeutel (2);
einem mit den Verarbeitungsbeutel (2) über eine Leitung (14) verbundenen Beutel (7) mit einer Nähr- und/oder Spüllösung,
einer dem Verarbeitungsbeutel (2) stromabwärts nachgeschalteten Hohlfasermembran-Filtereinheit (3); und
der Hohlfasermembran-Filtereinheit (3) stromabwärts nachgeschalteten Sammelbeuteln (4, 5, 6) zumindest für eine Erythrozyten/Plasma-Suspension und eine von der Filtereinheit abgetrennte Plasmafraktion, wobei
der Sammelbeutel (4) für die Erythrozyten/Plasma-Suspension eine Fluidverbindung (13, 18) aufweist, welche die Erythrozyten/Plasma-Suspension aus dem Sammelbeutel (4) zu dem Verarbeitungsbeutel (2) zurückführt, sodass durch den Verarbeitungsbeutel (2), die Hohlfasermembran-Filtereinheit (3) und den Sammelbehälter (4) ein Kreislauf gebildet ist.

## Claims

1. A method for separating whole blood into at least two fractions under the influence of gravity by means of a hollow fiber membrane filtration unit (3), with the steps:
- splitting off thrombocytes and leukocytes from whole blood contained in a withdrawal bag (1) by means of a leukocyte filter, with erythrocytes and plasma remaining in suspension,
- feeding the erythrocyte/plasma suspension into a processing bag (2),
- splitting off cell-free plasma from the erythrocyte/plasma suspension in a first separation step, by feeding the erythrocyte/plasma suspension from the processing bag (2) through the hollow fiber membrane filtration unit (3) into collecting bags (4, 5, 6),
- suspending of the erythrocyte/plasma suspension obtained in this way in the processing bag (2) with a nutrient and/or rinsing solution under sterile conditions (7, 14, 19),
- splitting off cell-free plasma from the suspended erythrocyte/plasma suspension in a second separating step, by feeding the erythrocyte/plasma suspension in a circulation from the at least one collecting bag (4) for the erythrocyte/plasma suspension via a fluid connection (13) into the processing bag (2) and then again through the hollow fiber membrane filtration unit (3), wherein
- the erythrocyte/plasma suspension flows through the hollow fiber membrane filtration unit (3) in the same direction in both separating steps.

2. The method according to claim 1, **characterized in that** the optionally repeated recirculating feeding back (13, 18, 2) of the erythrocyte/plasma suspension to the inlet of the hollow fiber membrane filtration unit (3) takes place for a number of times until a fluidity limit of the concentrated erythrocytes in the residual plasma is reached.

3. The method according to claim 2, **characterized in that** the fluidity limit substantially corresponds to a hematocrit of about 90%.

4. The method according to claim 1, **characterized in that** the suspending by means of the nutrient and/or rinsing solution takes place before each renewed passage through the hollow fiber membrane filtration unit (3).

5. The method according to one of claims 1 to 4, **characterized in that** at least before one passage through the hollow fiber membrane filtration unit (3), at least one substance altering the cellular properties of the erythrocyte/plasma suspension in a desired manner is added to the erythrocyte/plasma suspension.

6. The method according to claim 5, **characterized in that** the at least one substance altering the cellular properties of the erythrocyte/plasma suspension is removed again in the hollow fiber membrane filtration unit (3).

7. The method according to one of claims 1 to 6, **characterized in that** the feeding back and in a given case the treatment of the erythrocyte/plasma suspension recovered in the first run takes place in a central treatment bag (2) arranged upstream of the hollow fiber membrane filtration unit (3).

8. Apparatus provided for carrying out the method according to one or several ones of claims 1 to 7, including:
a withdrawal bag (1) for receiving a whole blood preserve;
a leukocyte filter (1 a) arranged downstream from the withdrawal bag (1);
a processing bag (2) arranged downstream from the leukocyte filter (1 a);
a bag (7) with nutrient and/or rinsing solution connected to the processing bag (2) via a conduit (14)
a hollow fiber membrane filtration unit (3) arranged downstream of the processing bag (2); and
collecting bags (4, 5, 6) at least for an erythrocyte/plasma suspension and a plasma fraction separated out by the filtration unit which are arranged downstream from the hollow fiber membrane filtration unit (3), wherein
the collecting bag (4) for the erythrocyte/plasma suspension has a fluid connection (13, 18) which feeds back the erythrocyte/plasma suspension from the collecting bag (4) to the processing bag (2) so that the processing bag (2), the hollow fiber membrane filtration unit (3), and the collecting bag (4) form a circulation.

## Revendications

1. Procédé de séparation de sang entier en au moins deux fractions sous l'influence de la force de gravité à l'aide d'une unité de filtre à membrane de fibre creuse (3), avec les étapes :
- séparation des thrombocytes et des leucocytes du sang entier contenu dans une poche de prélèvement (1) à l'aide d'un filtre de leucocyte en laissant les érythrocytes et le plasma en suspension,
- conduite de la suspension d'érythrocytes et de plasma dans une poche de traitement (2),
- séparation du plasma sans cellule, de la suspension d'érythrocytes et de plasma dans une première étape de séparation, en ce que celle-ci est conduite de la poche de traitement (2) au travers de l'unité de filtre à membrane de fibre creuse (3) dans la poche collectrice (4, 5, 6),
- tenue en suspension de la suspension d'érythrocytes et de plasma ainsi obtenue dans la poche de traitement (2), avec une solution nutritive et/ou de rinçage dans des conditions stériles (7, 14, 19) ;
- séparation du plasma sans cellule, de la suspension d'érythrocytes et de plasma tenue en suspension dans une seconde étape de séparation en ce que celle-ci est conduite en circulation de l'au moins une poche collectrice (4) pour la suspension d'érythrocytes et de plasma par une liaison fluidique (13) dans la poche de traitement (2) et ensuite de nouveau au travers de l'unité de filtre à membrane de fibre creuse (3), dans lequel
- la suspension d'érythrocytes et de plasma traverse l'unité de filtre à membrane de fibre creuse (3) dans les deux étapes de séparation dans la même direction.

2. Procédé selon la revendication 1, **caractérisé en ce que** le retour (13, 18, 2) recirculant éventuellement de manière répétée de la suspension d'érythrocytes et de plasma est effectué sur l'entrée de l'unité de filtre à membrane de fibre creuse (3) aussi souvent qu'une limite de fluidité des érythrocytes épaissis est atteinte dans le plasma restant.

3. Procédé selon la revendication 2, **caractérisé en ce que** la limite de fluidité correspond sensiblement à un hématocrite d'environ 90 %.

4. Procédé selon la revendication 1, **caractérisé en ce que** la tenue en suspension est effectuée avec la solution nutritive et/ou de rinçage avant chaque nouveau passage de l'unité de filtre à membrane de fibre creuse (3).

5. Procédé selon l'une des revendications 1 à 4, **caractérisé en ce qu'**au moins avant un passage de l'unité de filtre à membrane de fibre creuse (3), au moins une substance modifiant de manière souhaitée les propriétés cellulaires de la suspension d'érythrocytes et de plasma est ajoutée à la suspension d'érythrocytes et de plasma.

6. Procédé selon la revendication 5, **caractérisé en ce que** l'au moins une substance modifiant les propriétés cellulaires de la suspension d'érythrocytes et de plasma est de nouveau retirée dans l'unité de filtre à membrane de fibre creuse (3).

7. Procédé selon l'une des revendications 1 à 6, **caractérisé en ce que** le retour et éventuellement le traitement de la suspension d'érythrocytes et de plasma obtenue dans le premier passage sont effectués dans une poche de traitement centrale (2) qui est montée en amont de l'unité de filtre à membrane de fibre creuse (3).

8. Dispositif prévu pour la réalisation du procédé selon l'une ou plusieurs des revendications 1 à 7, présentant :
une poche de prélèvement (1) pour la réception d'une poche de sang entier ;
un filtre de leucocytes (1a) monté en aval de la poche de prélèvement (1) ;
une poche de traitement (2) montée en aval du filtre de leucocytes (1a) ;
une poche (7) reliée à la poche de traitement (2) par une conduite (14) avec une solution nutritive et/ou de rinçage,
une unité de filtre à membrane de fibre creuse (3) montée en aval de la poche de traitement (2) ; et
des poches collectrices (4, 5, 6) montées en aval de l'unité de filtre à membrane de fibre creuse (3) au moins pour une suspension d'érythrocytes et de plasma et une fraction de plasma séparée de l'unité de filtre, dans lequel
la poche collectrice (4) pour la suspension d'érythrocytes et de plasma présente une liaison fluidique (13, 18) qui ramène la suspension d'érythrocytes et de plasma depuis la poche collectrice (4) à la poche de traitement (2) de sorte qu'un circuit soit formé par la poche de traitement (2), l'unité de filtre à membrane de fibre creuse (3) et le récipient collecteur (4).
